**Europäisches Patentamt**

**European Patent Office**

**Office européen des brevets**

(11) Veröffentlichungsnummer: **0 280 644**
**A2**

# EUROPÄISCHE PATENTANMELDUNG

(21) Anmeldenummer: **88730043.2**

(22) Anmeldetag: **26.02.88**

(51) Int. Cl.⁴: **A 61 N 1/16**

(30) Priorität: **26.02.87 DE 3706589**

(43) Veröffentlichungstag der Anmeldung:
**31.08.88  Patentblatt  88/35**

(84) Benannte Vertragsstaaten:
**AT BE CH DE FR GB LI NL SE**

(71) Anmelder: **Schumacher, Hanns-Joachim, Dr. med. dent.**
**Millöckerweg 8a**
**D-2000 Hamburg 73  (DE)**

(72) Erfinder: **Schumacher, Hanns-Joachim, Dr. med. dent.**
**Millöckerweg 8a**
**D-2000 Hamburg 73  (DE)**

(74) Vertreter: **Patentanwälte Wenzel & Kalkoff**
**Grubes Allee 26 Postfach 730466**
**D-2000 Hamburg 73  (DE)**

(54) **Anordnung zum Ermitteln und/oder Beeinflussen von elektromagnetischen Wellen, Verfahren zur Herstellung der Anordnung sowie Antenne zum Ausführen des Verfahrens und zum Aufbau der Anordnung.**

(57) Anordnung (71) zum Ermitteln und/oder Beeinflussen von elektromagnetischen Raumwellen und/oder einer Gruppe solcher Wellen, Verfahren zur Herstellung der Anordnung (71) sowie Antenne (1) zum Ausführen des Verfahrens und zum Aufbau der Anordnung (71). Um elliptisch- und/oder zirkularpolarisierte Wellen mit einer frequenzunabhängig wirksamen Antennenanordnung hinsichtlich des Polarisationsdrehsinns zu beeinflussen, wird mindestens eine frequenzunabhängige Antenne (1) mit ihrem Zentrum (13) in einer elektromagnetischen Phase/Strahlungsfront (200) von zirkular- oder elliptisch-polarisierten Wellen höchster Rauschintensität pro Raumvolumenheit angeordnet und ausgerichtet. Die Antenne umfaßt mindestens zwei um eine Strahlungsachse sich öffnende, im Abstand und isoliert zueinander angeornete Spiralarme, deren innere Enden (131, 132) im Antennenzentrum (13) liegen und mit jeweils einem kreis- oder kugelförmigen Dipolelement (133, 134) verbunden sind.

Fig.2

EP 0 280 644 A2

## Beschreibung

Anordnung zum Ermitteln und/oder Beeinflussen von elekmagnetischen Wellen, Verfahren zur Herstellung der Anordnung sowie Antenne zum Ausführen des Verfahrens und zum Aufbau der Anordnung

Die Erfindung betrifft eine Anordnung zum Ermitteln und/oder Beeinflussen von elektromagnetischen Raumwellen und/oder einer Gruppe solcher Wellen, die von Sendern oder Strahlern wie Mikrowellen erzeugenden Einrichtungen, Körpern, Resonanzquellen oder -strukturen, z.B. geologischen Spalten, Strömungen fließender Medien (z.B. Wasser) od.dgl. abgestrahlt werden, ein Verfahren zur Herstellung der Anordnung sowie eine Antenne zum Ausführen des Verfahrens und zum Aufbau der Anordnung.

Elektromagnetische Wellen, die von den genannten Sendern oder Strahlern ausgehen, weisen eine elliptische oder zirkulare Polarisation auf. In Ausbreitungsrichtung der Welle gesehen, dreht sich an einem festen Ort der elektrische Feldvektor abhängig von der Zeit im Drehsinn der Polarisation. Durch eine (gedachte) Verbindung der Pfeilspitzen der elektrischen Feldvektoren in der sich ausbreitenden Welle erhält man eine schraubenförmige Linie, deren Drehsinn entgegengesetzt zu dem Polarisationsdrehsinn des Feldstärkevektors ist.

Elliptisch- oder zirkularpolarisierte elektromagnetische Wellen werden insbesondere durch die Bewegung elektrisch geladener Teilchen in einem Magnetfeld verursacht. Sie werden von Mikrowellenstrahlern wie Richtstrahlern und Satellitenantennen sowie auch von Einrichtungen oder technischen Geräten abgestrahlt, die Magnete aufweisen bzw. während des Betriebs elektromagnetische Felder erzeugen. Als technische Geräte dieser Art sind insbesondere Kathodenstrahl-Bildschirm-/Monitorgeräte und elektromagnetische Wechselstromanlagen wie Drehstrommaschinen, Wechselstromleitungen und Wechselstromgeneratoren zu nennen. Weiterhin ist es bekannt, daß elektromagnetische elliptisch-/zirkular-polarisierte Wellen im Zusammenwirken mit globalen oder kosmischen Feldern und elektrisch geladenen Teilchen von Resonanzquellen oder -strukturen wie geologischen Spalten und Strömungen fließender Medien, z.B. unterirdischen Wasserläufen ausgehen. Sie weisen Wellenlängen im Bereich von mehreren Metern bis hin zu Millimetern und darunter auf.

Empfangssysteme von elektromagnetischen zirkular-polarisierten Richtstrahlen oder deren Fernfelder können durch zirkular-/elliptisch-polarisierte Wellen anderer Sender oder Strahler gestört werden. Um diese unerwünschte sogenannte Kreuzpolarisation zu vermeiden, müssen die sie erzeugenden Feldkomponenten der störenden elliptisch-/zirkular-polarisierten Wellen gelöscht werden.

Die elektromagnetischen elliptisch-/zirkular polarisierten Wellen im Hoch- und/oder Höchstfrequenzbereich können für Menschen vorteilhaft oder schädlich sein, wie dies durch Bedeutung und Wirkung von Polarisationsvorgängen bei biochemischen Stoffen indiziert ist. Es ist die Molekularspektroskopie zu erwähnen, in der spezifische Frequenzen mit besonders hohen Intensitäten gemessen worden sind, z.B. Linienfrequenzen von $H_2$, OH, $H_2O$, HCOOH (Ameisensäure) u.a.. Die Polarisation der elektromagnetischen Wellen ist ein typisches Kennzeichen des biologischen Lebens. So bestimmt bei natürlich erzeugten organischen Verbindungen, wie z.B. links- und rechts drehender Milchsäure, die optische Polarisationsdrehung die biologische Wirkung im Lebewesen. Bei biochemischen Reaktionen wie z.B. der Photosynthese entstehen Reaktionsprodukte, die die Energiequelle für biochemische Umsetzungen in Pflanzen und Tieren sind. Weiterhin sei erwähnt, daß die biologische Wirksamkeit von zirkular-polarisierten Wellen bei Untersuchungen der die Gene bildenden DNS-Doppelspirale nachgewiesen worden ist. Daher ist davon auszugehen, daß das Vorhandensein der Strahlung und deren Polarisationsdrehsinn auf den menschlichen Organismus einwirken und folglich auch Beschwerden oder Krankheiten verursachen und/oder begünstigen können. In Anbetracht dieser Erkenntnisse ist es wünschenswert, elektromagnetische elliptisch-/zirkular-polarisierte Wellen einfach zu ermitteln und auch deren ggf. für die Gesundheit des Menschen schädliche Wirkung zu vermeiden.

Eine bekannte Vorrichtung zur Beeinflussung von sogenannten "terrestrischen Strahlen" (DE-OS 34 18 426) umfaßt eine Antenne mit einem Rohr und mit im rechten Winkel daran angeordneten Antennenstäben. Die Strahlung der Antenne erfolgt ausschließlich in Richtung eines am oberen Ende des Rohres angeordneten Permanentmagneten, so daß sie nur an dem oberen Ende abstrahlen kann. Die Anordnung eines oder mehrerer Antennenstäbe in verschiedenen, senkrecht zu dem Rohr verlaufenden Ebenen sowie ggf. die Anordnung von Winkelabständen zwischen den Antennenstäben können zwar zu einem Einwirken auf elektromagnetische linear-polarisierte Wellen führen, nicht jedoch zu einer wirksamen Beeinflussung von elliptisch-/zirkular-polarisierten Feldkomponenten. Bei einer anderen bekannten Vorrichtung (DE-GM 83 33 190.5) ist eine Schrauben-/Wendelantenne vorgesehen, die aus Kupfer besteht und aufgrund der diamagnetischen Eigenschaft dieses Materials zu einem Auslöschen von unterirdischen elektromagnetischen Wellen führen soll. Die horizontale Charakteristik der Antenne ist nicht geeignet, elektromagnetische elliptisch-/zirkular-polarisierte Wellen in einem ausreichenden Maß zu beeinflussen. An Oberflächen des den Antennenwendel bildenden Kupferdrahtes entstehen aufgrund des Skin-Effekts elektrostatische Aufladungen, die einer zufriedenstellenden Arbeitsweise der Antenne, die für eine bestimmte Frequenz ausgelegt sein muß, entgegenstehen. Auch eine bekannte Vorrichtung mit mindestens einem metallischen Bügel und ggf. Umwicklung mindestens eines Bügelschenkels mit einem elektrisch leitenden Draht (DE-GM 84 30 355.7) ist nicht geeignet, elektromagnetische elliptisch-/zirkular-polarisierte Wellen zu beeinflussen. Die Schenkel des Bügels sowie auch der ggf. sie umgebende Draht wirken als Dipole, wobei die Gesamtcharakteristik der Antennenvorrichtung nur linear-polarisierte Wellen abstrahlen kann. Bei einem noch anderen Gerät (DE-AS 35 01 532) sollen radiästhetische Strahlen entfernt werden. Es können nur linear-polarisierte Strahlen beeinflußt werden. Zudem sind Vorrichtungen bekannt, die die Abschirmung von elektromagnetischen Feldern betreffen (DE-OS 31 31 137). In Beton eingebettete Fasern sind als Dipole vorgesehen. Spiralförmige Fasern bilden einen Dipol, sind frequenzabhängig und liegen ungeordnet im Beton, so daß eine Beeinflussung von elliptisch-/zirkularpolarisierten Wellen insbesondere in einem breiten Frequenzbereich nicht möglich ist.

Der Erfindung liegt die Aufgabe zugrunde, elektromagnetische elliptisch- und/oder zirkular-polarisierte Wellen mit einer frequenzunabhängig wirksamen, einfach herstellbaren und installierbaren handlichen Antennenanordnung gezielt hinsichtlich des Polarisationsdrehsinns zu beeinflussen.

Bei einer erfindungsgemäßen Anordnung wird die Aufgabe dadurch gelöst, daß mindestens eine Antenne, die als frequenzunabhängiger Strahler ausgebildet ist, wenigstens zwei um eine Antennen-Zentrum- oder Strahlungsachse sich sämtlich jeweils links- bzw. rechtsdrehend öffnende, im Abstand und isoliert zueinander angeordnete spiralförmige Abschnitte oder Arme für elektromagnetische zirkular- oder elliptisch-polarisierte Wellen sowie kugelflächen- oder kreisflächenförmige, mit im Bereich des Antennenzentrums liegenden inneren Spiralabschnittenden verbundene Antennen-Dipolabschnitte aufweist, mit ihrem Antennenzentrum in einer elektromagnetischen Phasen-/Strahlungsfront von zirkular- oder elliptisch polarisierten Wellen oder Gruppen von Wellen mit höchster Rauschintensität pro Raumvolumeneinheit angeordnet ist. Zum Herbeiführen oder Aufbau der Antennenanordnung ist erfindungsgemäß vorgesehen, daß als Empfangssystem ein solches für elektromagnetische zirkular- oder elliptischpolarisierte Wellen verwendet wird, mit dem Empfangssystem eine elektromagnetische Phasen- oder Strahlungsfront mit höchster Rauschintensität pro Raumvolumeneinheit aufgesucht wird, als Antenne eine solche mit großer Bandbreite verwendet wird, die mindestens zwei um eine Antennen-Zentrum- oder Strahlungsachse sich sämtlich jeweils links-bzw. rechtsdrehend öffnende, im Abstand und isoliert zueinander angeordnte spiralförmige Abschnitte oder Arme für elektromagnetische zirkular- oder elliptisch-polarisierte Wellen sowie kugelflächen- oder kreisflächenförmige, mit im Bereich des Antennenzentrums liegenden inneren Spiralabschnittenden verbundene Antennen-Dipolabschnitte aufweist, wenigstens eine Antenne derart angeordnet und ausgerichtet wird, daß das Antennenzentrum in einer Phasen-/Strahlungsfront mit höchster Rauschintensität zu liegen kommt, und anschließend die Antenne durch Drehung um das Antennenzentrum und/oder Verschiebung des Antennenzentrums entlang einer Phasen-/Strahlungsfront mit höchster Rauschintensität in eine die elektromagnetische zirkular- oder elliptisch-polarisierte Raumwellen oder Raumwellengruppe beeinflussende Position gebracht wird. Eine erfindungsgemäße Antenne für den Aufbau einer Anordnung und/oder die Ausführung eines dafür vorgesehenen Verfahrens umfaßt mindestens zwei um eine Antennen-Zentrum-oder -strahlungsachse sich sämtlich jeweils links- bzw. rechtsdrehend öffnende, im Atstand und isoliert zueinander angeordnete Spiralarme, deren innere Spiralarmenden im Bereich des Antennenzentrums liegen, wobei mit mindestens zwei inneren Spiralarmenden jeweils ein kreis- oder kugelförmiges Dipol-Antennenelement zur Ausbildung einer mit der Spiralarmantenne integrierten Dipolantenne verbunden ist.

Die elektromagnetischen elliptisch-/zirkular-polarisierten Wellen eines Senders oder Strahlers werden durch die Anordnung mindestens einer Antenne nach Maßgabe ihrer Charakteristik und Ausrichtung empfangen und nach Beeinflussung abgestrahlt. Man erhält phasenverschobene resultierende Wellen, deren elliptisch-/zirkular-polarisierte Feldkomponenten hinsichtlich des Polarisationsdrehsinns beeinflußt oder verändert sind. Damit können unerwünschte Kreuzpolarisationen in Richtstrahler-Empfangssystemen und/oder in Fernfeldern von Richtstrahlern unterdrückt oder beseitigt werden. Die Beeinflussung des Polarisationsdrehsinns führt zu resultierenden Wellen mit veränderten medizinisch-biochemischen Wirkungen, so daß auch für die Gesundheit des Menschen schädliche Wirkungen beseitigt sowie ggf. der Gesundheit förderliche Wirkungen hervorgebracht und nutzbar gemacht werden können. Die Ausbildung der spiralförmigen Abschnitte in Kombination mit an die inneren Spiralabschnittenden angekoppelten Antennen-Dipolabschnitten gewährleistet eine frequenzunabhängige Antenne mit ausgeprägter elliptischer-/zirkularer-Empfangs-/Strahlungscharakteristik in mindestens zwei Antennenebenen. Dabei baut die wenigstens eine Antenne der Anordnung sehr einfach, so daß das Antennenzentrum zur optimalen Wirkung auf die zu beeinflussenden Wellen bequem in einer elektromagntischen Phasen-/Strahlungsfront oder in einem Bereich zirkular-oder elliptisch-polarisierter Wellen oder einer Gruppe von solchen Wellen mit höchster Rauschintensität pro Raumvolumeneinheit positionierbar ist. Eine elektromagnetische Phasen- oder Strahlungsfront mit höchster Rauschintensität kann schnell und wirksam mit einem Empfangssystem für elektromagnetische zirkular- oder elliptischpolarisierte Wellen aufgesucht werden. Nachdem das Antennenzentrum in eine Phasen-/Strahlungsfront gebracht worden ist, erfolgt eine gezielte Beeinflussung der von einem Sender oder Strahler ausgehenden elektromagnetischen zirkular-/elliptisch-polarisierten Wellen auf einfache Weise dadurch, daß die Antenne um ihr Zentrum gedreht und/oder das Zentrum entlang der Phasen-/Strahlungsfront mit höchster Rauschintensität verschoben wird. Die erfindungsgemäße Anordnung ist stationär eingerichtet und erlaubt ein andauerndes, durch Interferenz bewirktes Auslöschen von zirkular-/elliptisch-polarisierten Feldkomponenten.

Bei zu beeinflussenden Wellen von Sendern oder Strahlern mit rotationssymmetrischer Strahlung erreicht man eine sehr wirksame Beeinflussung dadurch, daß eine senkrecht zu der Antennen-Zentrumachse liegende Antennenfläche quer, insbesondere senkrecht zu einer Fortpflanzungsrichtung der zirkurlar-/elliptisch-polarisierten Wellen angeordnet ist. Auf Wellen nicht-rotationssymmetrischer Strahler, d.h. von Sendern mit Richtwirkung und/oder einer sich ggf. in Linienrichtung fortpflanzenden strahlenden Linie, erfolgt die Beeinflussung auf einfache Weise gezielt und wirksam dadurch, daß die Anordnung eine Antenne umfaßt, deren äußere Spiralabschnittenden auf einer Antennenlängsachse münden, wobei diese im wesentlichen parallel zu der strahlenden Linie und/oder einer Längsachse des Senders ausgerichtet ist.

Mit der erfindungsgemäßen Ancrdnung können insbesondere linksschraubige, d.h. mit ihrem elektrischen Feldvektor rechtsdrehend zirkular-/elliptisch-polarisierte Wellen derart beeinflußt werden, daß zirkular-/elliptisch-polarisierte Feldkomponenten in einem dem Sender abgewandten Strahlungsbereich der Antenne durch Interferenz gelöscht sind. Zu diesem Zweck werden die Antennenabschnitte, in Fortpflanzungsrichtung der Phasen-/Strahlungsfront gesehen, im Gegen-Uhrzeigersinn sich öffnend angeordnet. Mit Antennenabschnitten, die in Fortpflanzungsrichtung der Phasen-/Strahlungsfront gesehen im Uhrzeigersinn sich öffnend angeordnet sind, können entsprechend zirkular-/elliptisch-polarisierte Feldkomponenten von (rechtsschraubigen) Wellen mit negativem Polarisationsdrehsinn des elektrischen Feldvektors gelöscht werden.

Eine besonders wirksame und vorteilhafte Antennenanordnung zum Löschen zirkular-/elliptisch-polarisierter Feldkomponenten durch Interferenz umfaßt Antennenspiralabschnitte, die beidseitig quer zu der Antennen-Zentrumachse mit einem zu den Abschnitten isolierten, linear-polarisierte Wellen reflektierenden Material, vorzugsweise aus Metall wie z.B. Aluminium abgedeckt sind. Dabei werden die linear-polarisierten Wellenkomponenten reflektiert, so daß sie zu keiner Neubildung der gelöschten Feldkomponenten mit Polarisationsdrehsinn beitragen können.

Eine besondere Gestaltungsart einer erfindungsgemäßen Anordnung besteht darin, daß sie zwei gleiche, die strahlende Linie eines Senders umgebende Antennen umfaßt, wobei diese jeweils mit ihrer zu der Antennen-Zentrumachse senkrechten Antennenfläche parallel zueinander ausgerichtet sind, sich jeweils mit einer in der Antennenfläche liegenden Antennenlängsachse entlang der strahlenden Linie erstrecken und wobei jeweils die Antennen-Spiralabschnitte, von dem Sender aus gesehen, im Gegen-Uhrzeigersinn sich öffnend ausgerichtet und auf den dem Sender abgewandten Antennenseiten mit einem zu den Spiralabschnitten isolierten linear-polarisierte Wellen reflektierenden Material abgedeckt sind.

Hinsichtlich eines besonders schnellen Aufbaus der Anordnung kann es vorteilhat sein, daß die Anordnung einen mindestens eine Antenne lagernden Tragkörper, wie ein Lager, ein Gehäuse, ein Gestell oder einen Rahmen umfaßt, an und/oder mit dem eine Antenne um ihr Zentrum drehbar und/oder entlang wenigstens einer Antennenachse verschiebbar angeordnet ist.

Zur Duchführung des erfindungsgemäßen Verfahrens wird besonders vorteilhaft ein an sich bekanntes Empfangssystem mit rauscharmem Empfänger und mit rauscharmer Antenne, insbesondere mit einer im Antennenzentrum eine Wendelantenne aufweisenden Rotationsparabolantenne verwendet. Die Rückseite der Rotationsparabolantenne wird vorzugsweise mit einem Mikrowellen absorbierenden Material wie rußhaltigem Schaumstoff abgedeckt. In Fällen, bei denen Rauschintensitäten technischer Geräte und der Empfang von Rauschleistung über Antennendiagramm-Nebengipfel relativ groß sind, ist es zweckmäßig, daß zum Aufsuchen von elektromagnetischen zirkular-/elliptisch-polarisierten Wellen für das Empfangssystem eine an sich bekannte, auf das menschliche Nerven-/Muskelsystem wirkende Hoch- oder Höchstfrequenzantenne, insbesondere eine V-förmige Antenne oder eine Lecher-Antenne mit Kurzschlußschieber verwendet wird, die insbesondere über einem Nord- oder Süd-Pol eines Magneten oder über dem Plus-oder Minus-Pol einer Batterie zur Feststellung einer Empfangsebene für ein magnetisches Feld bzw. für ein elektrisches Feld geeicht wird.

Eine besonders geeignete Antenne zum Aufbau der Anordnung bzw. zur Durchführung des Verfahrens ist so ausgebildet, daß die im Bereich des Antennenzentrums liegenden inneren Spiralarmenden von einer Antennenquerachse ausgehen, die im Antennenzentrum senkrecht auf der Antennen-Zentrumachse steht. Eine zweckmäßige und besondere Richt- und Abstrahlcharakteristik der Antenne läßt sich vorteilhaft auch dadurch erreichen, daß äußere Spiralarmenden auf einer Antennenlängsachse enden, die im Antennenzentrum senkrecht auf der Antennen-Zentrumachse steht. Besonders vorteilhaft ist es, daß die Antennenlängsachse senkrecht zu der Antennenquerachse verläuft. Mit dieser Gestaltung läßt sich die Antenne insbesondere bei nicht-rotationssymmetrischen Sendern oder Strahlern sehr gezielt und definiert mit besonders guter Wirkung in eine die Sender-/Strahlerwellen beeinflussende Position bringen.

Zur Beeinflussung von Sender-Wellen mit Frequenzen im Gigahertzbereich ist es vorteilhaft, daß die Spiralarme und/oder die Dipol-Antenneneemente aus einem einen elektrischen Widerstand aufweisenden (nicht-metallischen) Material, insbesondere aus Kohlenstoff oder Graphit bestehen. Insbesondere durch weiche und/oder harte kosmische Strahlung können sich Metallabschnitte durch Ionenbildung elektrostatisch aufladen. Eine solche Aufladung ist unerwünscht, da die Antenne damit einen nicht beabsichtigten Dipolcharakter erhält, der ihre Eignung, zirkular-/elliptisch-polarisierte elektromagnetische Wellen zu beeinflussen, empfindlich beeinträchtigen kann.

Besonders vorteilhaft ist es, eine Antenne mit einer geradzahligen Anzahl von Dipolelementen und Spiralarmen auszubilden. Eine Antenne mit zwei Spiralarmen und einem im Antennenzentrum konzentrierten Dipol, der aus zwei jeweils mit dem zugehörigen Spiralarmende verbundenen Dipol-Antennenelementen besteht, weist die für die Zwecke der Erfindung erforderliche Charakteristik auf, ist besonders einfach herstellbar und sehr kleinbauend ausführbar. Auch ist es vorteilhaft, daß die Spiralarme mit ihren Dipol-Antennenelementen Bestandteil eines blattförmigen Antennenkörpers sind. Eine solche Antenne weist vorteilhaft eine Flächengröße auf, die 600 mm² beträgt oder noch erheblich kleiner ist. Der Antennenkörper ist zweckmäßig mit einer abziehbaren, eine Haft-oder Klebeschicht freilegenden Abdeckschicht oder Folie versehen, um die Antenne zur Ausbildung der Anordnung besonders schnell und dauerhaft positionieren zu können.

Für die Zwecke der Erfindung sind besonders Antennen mit Spiralarmen geeignet, deren Durchmesser oder Dicke in Sprialöffnungsrichtung zunehmend ausgebildet ist. Eine Antenne mit derartiger Gestalt der Spiralarme ist zweckmäßig als logarithmische Spiralantenne ausgebildet.

Eine erfindungsgemäße Antenne zum besonders wirksamen Löschen von zirkular-/elliptisch-polarisierten Feldkomponenten weist in vorteilhafter Ausgestaltung eine die Spiralarme sowie die Dipol-Antennenelemente quer zu der Antennen-Zentrumachse beidseitig abdeckende Schicht auf, die aus einem linear-polarisierte Wellen reflektierenden Material, vorzugsweise aus einem Metall wie Aluminium gebildet ist. Eine Antenne mit Spiralarmen und DipolAntennenelementen aus nicht-metallischem Material ist unmittelbar in Metallfolie eingewickelt oder von dieser umgeben.

Weitere Ausführungsarten der Erfindung gehen aus den Unteransprüchen hervor.

Die erfindungsgemäße Anordnung, das erfindungsgemäß Verfahren zur Herstellung derselben sowie die erfindungsgemäße Antenne gewährleisten eine gezielte, sehr einfach durchführbare Auslöschung von zirkular-/elliptisch-polarisierten Feldkomponenten durch Interferenz.

Weitere Ausbildungsmöglichkeiten, Zweckmäßigkeiten und Vorteile der Erfindung gehen aus der folgenden Beschreibung der in der teilweise stark schematisierten Zeichnung dargestellten Ausführungsbeispiele hervor. In der Zeichnung zeigen

Fig. 1 ein Strahlungsdiagramm eines elektromagnetischen Strahlers,

Fig. 2 und 3 in Draufsicht und Seitenansicht eine erfindungsgemäße Antennenanordnung mit einer strahlenden unterirdischen Strömung,

Fig. 4 eine erfindungsgemäße Antennenanordnung,

Fig. 5 eine erfindungsgemäße Antennenanordnung mit zwei eine strahlende Strömung umgebenden Antennen und

Fig. 6 eine erfindungsgemäße Spiral-/Dipol-Antenne.

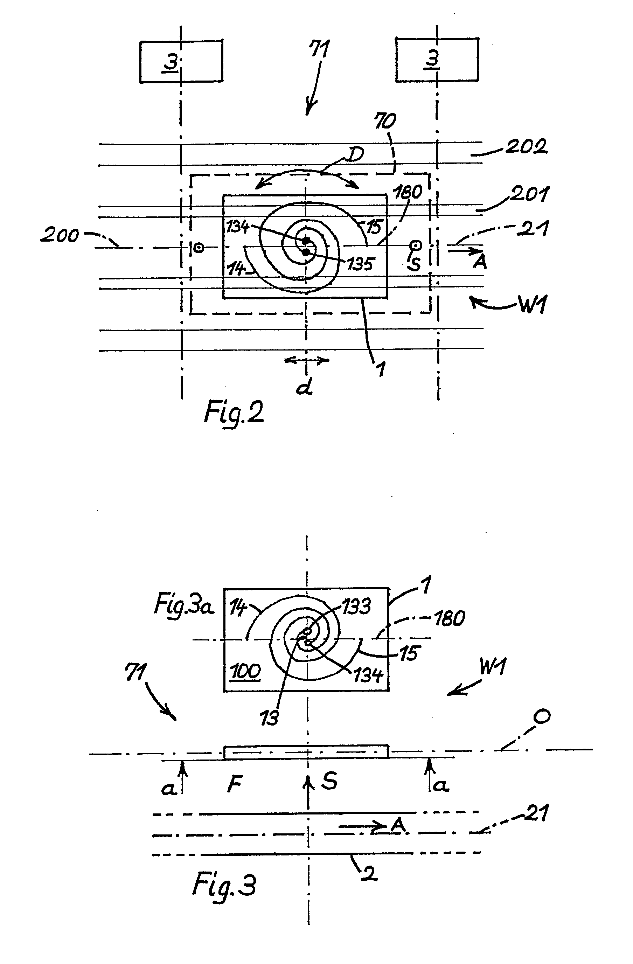

Ausführungsgeispiele erfindungsgemäßer Anordnungen 7 zum Ermitteln und/oder Beeinflussen von elektromagnetischen zirkular-/elliptisch-polarisierten Raumwellen und/oder einer Gruppe solcher Wellen sind in Fig. 2 bis 5 dargestellt. Anordnungen 71 und 72 umfassen jeweils eine einzige Spiral-/Dipol-Antenne 1, während für die Anordnung 73 zwei gleiche Spiral-/Dipol-Antennen 1 vorgesehen sind.

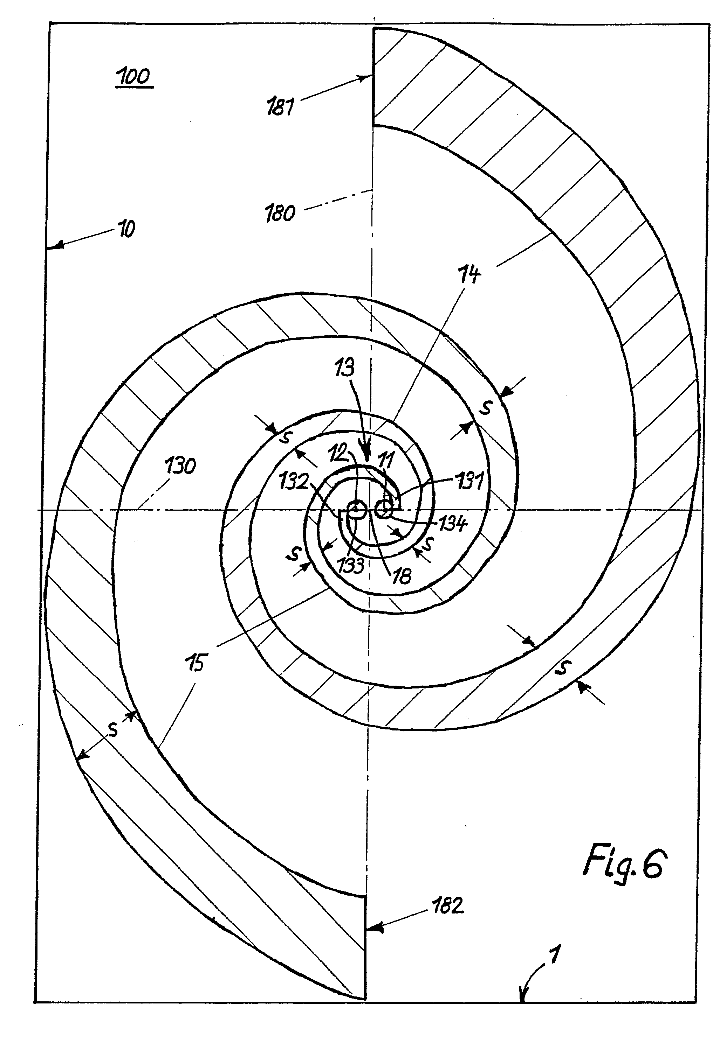

Aus Fig. 6 gehen Details einer Antenne 1 hervor. Flächig ausgebildete Spiralarme 14 und 15 sind in oder an einer Fläche 100 isoliert zueinander angeordnet. Sie öffnen sich zunehmend und linksdrehend um eine senkrecht zu der Antennenfläche 100 stehende Antennen-Zentrumachse 18. Innere Spiralabschnittenden 131 und 132 gehen von einer in der Fläche 100 liegenden Antennenquerachse 130 aus, die die Zentrumachse 18 im Antennenzentrum 13 senkrecht schneidet. Die Flächenbreite bzw. der Flächendurchmesser s jedes Spiralarms 14, 15 nimmt mit größer werdendem Spiralöffnungswinkel zu. Äußere Spiralabschnittenden 181 und 182 münden auf einer in der Fläche 100 liegenden und durch das Antennenzentrum 13 gehenden Antennenlängsachse 180.

Mit jedem inneren Spiralarmende 131, 132 ist jeweils ein kreis-flächenförmiges, in der Fläche 100 liegendes Dipol-Antennenelement 133 bzw. 134 mit parallel zu der Zentrumachse 18 liegenden Dipolachsen 11 und 12 verbunden. Die Dipolelemente sind mittig auf der Antennenquerachse 130 angeordnet, wobei sie zwischen dem Antennenzentrum 13 und den inneren Spiralarmenden 131, 132 seitlich an diese angekoppelt sind. Die Spiralarme 14, 15 sowie die Dipol-Antennenelemente 133, 134 bestehen aus einem einen elektrischen Widerstand aufweisenden Material wie insbesondere aus Kohlenstoff oder Graphit. Die für die Zwecke der Erfindung gewünschte Antennencharakteristik kann bereits dadurch erreicht werden, daß die in Fig. 6 schraffierten Flächen dunkel, insbesondere schwarz gefärbt sind.

Der Antennenkörper 10 ist insbesondere als dünne Blattträgerfläche ausgebildet, die vorzugsweise aus einem Blatt Papier besteht Die Antennenfläche 100 weist vor teilhaft eine Größe von ca. 15 x 20 mm2 auf, während sie aber auch noch beträchtlich kleiner ausgebildet sein kann. Eine solche Antenne ist sehr handlich. Sie läßt sich zur Durchführung des erfindungsgemäßen Verfahrens bzw. zum Aufbau der erfindungsgemäßen Anordnung besonders einfach handhaben und einsetzen. Sie ist zweckmäßig mit selbstklebenden Flächen ausgestattet, so daß sie an gewünschten Orten durch Ankleben an irgendwelche Gegenstände positioniert werden kann.

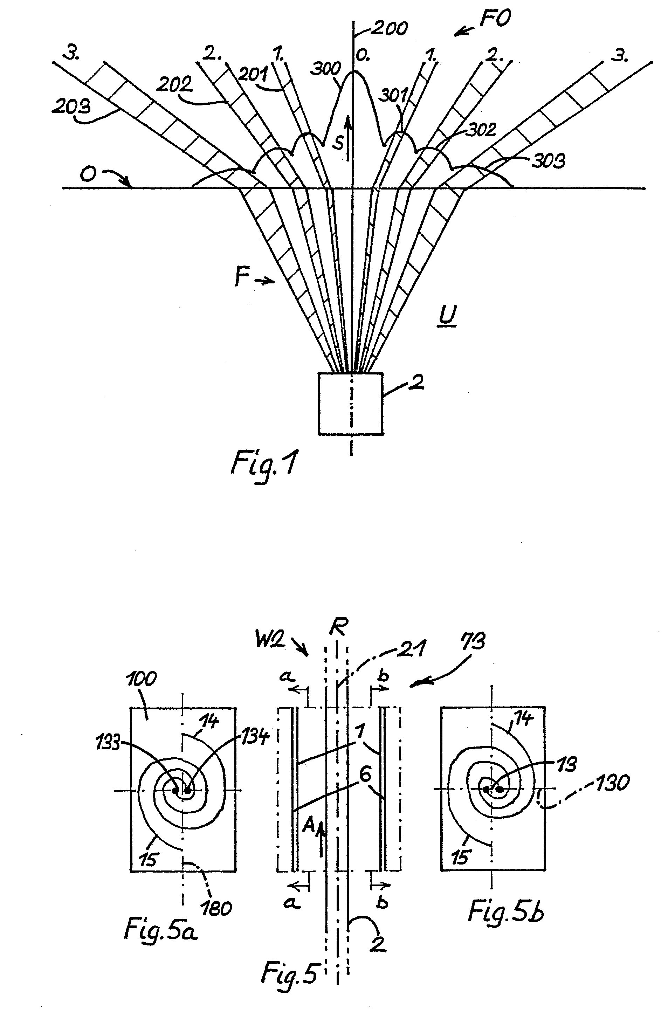

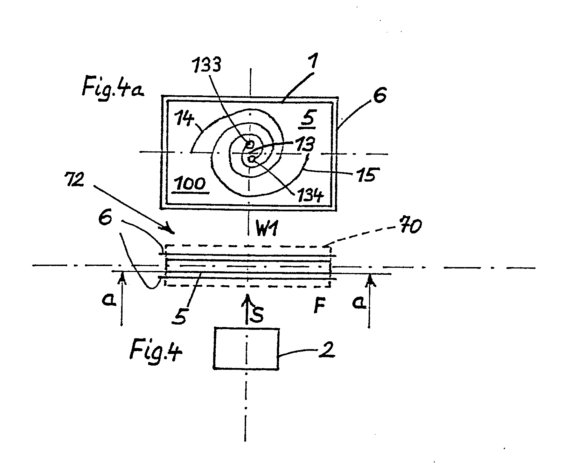

In dem Ausführungsbeispiel gemäß Fig. 4 umfaßt die Antenne 1 Schichten 6, die aus einem linear-polarisierte Wellen reflektierenden Material bestehen und den Antennenkörper 10 bzw. die Antennenfläche 100 beidseitig vollständig abdecken, wobei die Spiralarme 14, 15 und die Dipol-Antennenelemente 133, 134 zwischen den flächigen Schichten 6 eingebettet sind. Eine besondere Ausbildungsart einer solchen Antenne 1 besteht darin, daß ein Blatt, das mit Spiralarmen und Dipol-Antennenelementen aus einem einen hohen elektrischen Widerstand aufweisenden Material versehen ist, wenigstens einlagig in eine Aluminiumfolie eingewickelt ist.

Die Antennenanordnung 73 gemäß Fig. 5 umfaßt zwei im Abstand und parallel mit ihren Antennenflächen 100 ausgerichtete Antennen 1, wobei mittig und parallel zu den Antennen eine strahlende Sender-Linie 21 liegt. Jede Antenne 1 ist auf der der strahlenden Linie 21 abgewandten Seite flächig mit

einer linear-polarisierte Wellen reflektierenden Schicht 6, vorzugsweise mit einer Aluminiumfolie abgedeckt.

Die Antennenanordnung 71 (Fig. 2 und 3) ist in Verbindung mit einem unterirdischen, eine Richtstrahlcharakteristik aufweisenden Sender 2 aufgebaut. Ein solcher Sender kann z.B. durch einen unterirdischen Wasserlauf gebildet sein, der eine strahlende Linie 21 aufweist, wobei die Richtcharakteristik durch die Strömungsrichtung A bestimmt ist. Statt durch einen unterirdischen Wasserlauf kann der Sender 2 mit Richtcharakteristik insbesondere durch andere Resonanz- oder Hohlleitergebilde wie Flüssigkeit führende Rohre oder elektrische Leitungen gebildet sein, die sämtlich zirkular-/elliptisch-polarisierte elektromagnetische Wellen mit einer in Strömungsrichtung A aufweisenden Komponente abstrahlen.

An oder oberhalb einer (Erdober-) Fläche O auftretende elektromagnetische zirkular-/elliptisch-polarisierte Wellen oder Gruppen von Wellen werden anhand des Strahlendiagramms Fig. 1 erläutert. Von einem Sender 2 wird ein Feld F abgestrahlt, das einen (Untergrund-) Bereich U in Richtung auf die (Ober-) Fläche O durchdringt und von dieser in den Raum oberhalb derselben strahlt. Die Fläche O trennt ein optisch dichteres Medium im Bereich U von einem optisch dünneren Medium oberhalb der Fläche. Daher werden an der Fläche O elektromagnetische Strahlen gebrochen und in die dargestellten Richtungen vom Lot weg abgelenkt. Es sind Strahlen 0., 1., 2. und 3. Ordnung 200, 201, 202, 203 dargestellt, wobei der Strahl 0. Ordnung unabgelenkt bleibt. Er weist die größte Rauschintensität pro Raumvolumeneinheit auf, während die Rauschintensitäten von Strahlen höherer Ordnung mit zunehmender Ordnung abnehmen. Die unterschiedlichen Intensitäten sind mit. einer Hauptkeule 300 und Nebenkeulen 301, 302 und 303 dargestellt.

Zur Herstellung einer Antennenanordnung 7 wird zunächst mit einem rauscharmen Empfänger 3 (Fig. 2), der insbesondere eine rauscharme Rotationsparabolantenne mit einer im Antennenzentrum angeordneten Wendelantenne umfaßt, die Phasen-Strahlungsfront 200 mit höchster Rauschintensität, d.h. ein Strahl (Welle) oder auch mehrere Strahlen (Wellen) 0. Ordnung aufgesucht. Die Rückseite der Rotationsparabolantenne ist vorzugsweise mit einem Mikrowellen absorbierenden Material wie rußhaltigem Schaumstoff abgedeckt, um einen rückwärtigen Empfang von Nebenkeulen zu dämpfen. Weiterhin wird mit dem Empfänger 3 festgestellt, in welcher Ebene Maxima des elektromagnetischen Feldes auftreten. Die Antenne 1 wird dann mit ihrer Antennenfläche 100 senkrecht zu einer solchen Meßebene angeordnet. Dabei wird die Antenne so positioniert, daß ihr Antennezentrum 13 in der Phasen-/ Strahlungsfront 200 mit höchster Rauschintensität zu liegen kommt. In den Ausführungsbeispielen gemäß Fig. 2 bis 4 befindet sich die Antenne 1 mit ihrer Antennenfläche 100 in einer horizontalen, zu einer Wellen-Fortpflanzungsrichtung S senkrechten Richtung.

Mit der Antennenanordnung 71 gemäß Fig. 2 und 3 werden linksschraubig L, d.h. rechtsdrehend P zirkular-/elliptisch-polarisierte Wellen des Senders 2 mit strahlender Linie 21 beeinflußt. Um zirkular-/elliptisch-polarisierte Feldkomponenten in einem dem Sender 2 abgewandten und in Richtung A liegenden Strahlungsbereich WI durch Interferenzen zu löschen, werden die Antennenabschnitte 14 und 15, in Fortpflanzungsrichtung S der Phasen-/Strahlungsfront 200 gesehen, im Gegen-Uhrzeigersinn sich öffnend angeordnet. Da es sich bei dem Sender 2 der Anordnung 71 um ein zu beeinflussendes Feld mit einer eine Hauptkeule 300 (Strahl 0. Ordnung) aufweisenden Richtcharakteristik, also um ein nicht-rotationssymmetrisches Feld handelt, wird die Antennenlängsachse 180 parallel zu der strahlenden Linie 21 ausgerichtet. In dieser Antennenposition, die aus der Ansicht a-a (Fig. 3a) sowie aus der Draufsicht in Fig. 2 ersichtlich ist, sind die nicht gewünschten zirkular-/elliptisch-polarisierten Feldkomponenten des Senders 2 durch Interferenz gelöscht.

Die beschriebene optimale Positionierung der Antenne 1 wird durch Drehung D um das Antennenzentrum und/oder Verschiebung d desselben entlang der strahlenden Linie 21 erreicht. Zu diesem Zweck kann die Antenne an einem Tragkörper 70 angeordnet sein.

Die Antennenanordnung 72 gemäß Fig. 4 ist einem Sender 2 mit einem rotationssymmetrischen Wellenfeld zugeordnet. Die in linear-polarisierte Wellen reflektierendes Material 6 eingebettete bzw. von diesem umgebene Antenne 1 erweist sich als besonders geeignet, zirkular-/elliptisch-polarisierte Feldkomponenten in einem Bereich WI auf der dem Sender 2 abgewandten Antenneseite durch Interferenz zu löschen. Fig. 4a stellt eine Ansicht a-a der Fig. 4 dar. Anordnung und Orientierung der Spiralarme 14 und 15 erfolgen in der gleichen Weise wie bei der Antennenanordnung 71. Allerdings ist es bei dem Sender 2 mit rotationssymmetrischem Feld F nicht erforderlich, eine spezielle Drehausrichtung der Antenne 1 um das Antennenzentrum 13 vorzunehmen. - Die in linear-polarisierte Wellen reflektierendes Material 6 eingebettete Antenne 1 der Anordnung 72 kann ebenfalls zum Aufbau und zur Herstellung der Antennenanordnung 71 (Fig. 2 und 3) verwendet werden.

Die Antennenanordnung 73 (Fig. 5) ist vorgesehen, um aus zirkular-/elliptisch-polarisierten Wellen eines Senders 2, z.B. einer wasserführenden Leitung, mit strahlender Linie 21, die mit einer (Strömuns-)Richtung A eine Richtcharakteristik aufweist, Feldkomponenten mit positivem Polarisationsdrehsinn des elektrischen Feldvektors (linksschraubige Feldkomponenten) durch Interferenz zu löschen. Damit bleibt ein Feld W2 mit negativem Polarisationsdrehsinn (rechtsschraubige Wellen) übrig. Zu diesem Zweck ist ein Leitungsrohr des Senders 2 mittig zwischen zwei gleichen Antennen 1 angeordnet, die auf der dem Sender 2 angewandten Seite flächig mit einem linear-polarisierte Wellen reflektierenden Material 6 abgedeckt sind. Die Antennen 1 sind mit ihren Antennenflächen 100 parallel und symmetrisch zueinander ausgerichtet. Wie aus den Ansichten a-a (Fig. 5a) und b-b (Fig. 5b) ersichtlich, sind jeweils die Antennen-Spiralabschnitte 14 und 15, von dem Sender 2 ausgesehen, im Gegen-Uhrzeigersinn sich öffnend ausgerichtet. Die Antennenlängsachsen 180 erstrecken sich parallel zu der strahlenden Linie 21.

Die beschriebenen Verfahren und Anordnungen bzw. die Antenne 1 gemäß Fig. 6 beziehen sich auf die Beeinflussung von linksschraubig rechtsdrehend zirkular-/elliptisch-polarisierten Wellen bzw. Feldkomponenten eines Senders. Es ist ohne weiteres ersichtlich, daß zur Beeinflussung von rechtsschraubig linksdrehend zirkular/elliptisch polarisierten Wellen eines Senders eine Antenne bzw. Antennen mit, in Fortpflanzungsrichtung der Phasen-Strahlungsfront gesehen, im Uhrzeigersinn sich öffnend angeordneten Spiral-Antenneabschnitten vorgesehen werden.

Eine erfindungsgemäße Antennenanordnung zum Löschen von zirkular-/elliptisch-polarisierten Feldkomponenten umfaßt nach einer besonderen Ausbildungsart mehrere, z.B. sechs Antennen. Eine derartige Anordnung wird insbesondere vorgesehen, wenn Sender bildende Resonanzquellen, Hohlleiterstrukturen od.dgl. Wellen mit mehreren Hauptkeulen aufweisen. Dabei werden die Antennen insbesondere ohne Abstand aneinander folgend entweder nebeneinander mit parallelen Antennenlängsachsen 180 oder hintereinander mit parallelen Antennenquerachsen 130 angeordnet. Eine Art Matrix- oder Flächenmusteranordnung, d.h. ein Antennenfeld mit nebeneinander und hintereinander angeordneten Antennen, wird vorzugsweise in besonderer Ausbildung der Erfindung dann vorgesehen, wenn ein diffus, inhomo gen und/oder ungerichtet strahlendes (Gruppen-) Wellenfeld zu beeinflussen ist.

**Bezugszeichenliste**

1 Antenne
10 Antennenkörper
100 Antennenfläche
11 Dipolachse
12 Dipolachse
13 Antennenzentrum
130 Antennenquerachse
131 inneres Spiralabschnittende
132 inneres Spiralabschnittende
133 kugelflächen- oder
134 kreisflächen-förmiger Antennenabschnitt
14 Spiralarm, Öffnung im Gegen-Uhrzeigersinn
15 Spiralarm, Öffnung im Gegen-Uhrzeigersinn
18 Antennenzentrum-Achse
180 Antennenlängsachse
181 äußeres Spiralabschnittende
182 äußeres Spiralabschnittende
2 Sender
200 Phasen-/Strahlungsfront mit höchster Rauschintensität
201 Strahl 1. Ordnung
202 Strahl 2. Ordnung
203 Strahl 3. Ordnung
21 strahlende Linie
3 Empfangssystem
300 Hauptkeule

301 Nebenkeule
302 Nebenkeule
303 Nebenkeule
6 linear-polarisierte Wellen reflektierendes Material
7 Antennen-Anordnung im Wellenfeld
70 Rahmen
71 Anordnung mit Spiral-/Dipolantenne
72 Anordnung mit beidseitig abgedeckter Spiral-/Dipolantenne
73 Anordnung mit zwei nebeneinander angeordneten einseitig abgedeckten Spiral-/Dipolantennen
A Ausbreitungsrichtung eines Linienstrahlers
D Drehrichtung der Antenne
F Wellen-/Gruppenwellen-Feld
FO Oberflächen-Gruppen-/Wellen
L linksschraubige Welle
O Oberfläche
P rechtsdrehender elektrischer Feldvektor (positiver Drehsinn)
S Fortpflanzungsrichtung der Phase-/Strahlungsfront mit höchster Rauschintensität
U Untergrundbereich
W1 Wellenfeld-Bereich
W2 Wellenfeld-Bereich
d Verschiebung
s Spiralarmflächendicke, -durchmesser

**Patentansprüche**

1. Anordnung zum Ermitteln und/oder Beeinflussen von elektromagnetischen Raumwellen und/oder einer Gruppe solcher Wellen (F, FO), die von Sendern oder Strahlern (2) wie Mikrowellen erzeugenden Einrichtungen, Körpern, Resonanzquellen oder -strukturen, z.B. geologischen Spalten, Strömungen fließender Medien (z.B. Wasser) od.dgl. abgestrahlt werden, mit einer zum Empfang der Wellen ausgerichteten Antenne, **dadurch gekennzeichnet,** daß mindestens eine Antenne (1), die als frequenzunabhängiger Strahler ausgebildet ist, mindestens zwei um eine Antennen-Zentrum- oder Strahlungsachse (18) sich sämtlich jeweils links- bzw. rechtsdrehend öffnende, im Abstand und isoliert zueinander angeordnete spiralförmige Abschnitte oder Arme (14, 15) für elektromagnetische zirkular- oder elliptischpolarisierte Wellen sowie kugelflächen- oder kreisflächenförmige, mit im Bereich des Antennenzentrums (13) liegenden inneren Spiralabschnittenden (131, 132) verbundene Antennen-Dipolabschnitte (133, 134) aufweist, mit ihrem Antennenzentrum (13) in einer elektromagnetischen Phasen/Strahlungsfront (200) von zirkular- oder elliptisch polarisierten Wellen oder Gruppen von Wellen mit höchster Rauschintensität pro Raumvolumeneinheit angeordnet ist.

2. Anordnung nach Anspruch 1, **dadurch gekennzeichnet,** daß eine senkrecht zu der Antennen-Zentrumachse (18) liegende Antennenfläche (100) quer, insbesondere senkrecht zu einer Fortpflanzungsrichtung (S) der zirkular-/elliptisch-polarisierten Gruppen-/Wellen (F) angeordnet ist.

3. Anordnung nach Anspruch 1 oder 2, **dadurch gekennzeichnet,** daß sie eine Antenne (1) umfaßt, deren äußere Sprialabschnittenden (181, 182) auf einer Antennenlängsachse (180) münden, und daß die Antennenlängsachse im wesentlichen parallel zu einer strahlenden Linie (21) und/oder einer Längsachse des Senders (2) ausgerichtet ist.

4. Anordnung nach einem der Ansprüche 1 bis 3, **dadurch gekennzeichnet,** daß zur Beeinflussung von (linksschraubig (L)) rechtsdrehend (P) zirkular-/elliptisch-polarisierten Gruppen-/Raumwellen (F, FO) des Senders (2) die Antennenabschnitte (14, 15), in Fortpflanzungsrichtung (S) der Phasen-/Strahlungsfront gesehen, im Gegen-Uhrzeigersinn sich öffnend angeordnet sind, wobei zirkular-/elliptisch-polarisierte Feldkomponenten in einem dem Sender (2) abgewandten Strahlungsbereich (WI) der Antenne (1) gelöscht sind.

5. Anordnung nach einem der Anprüche 1 bis 3, **dadurch gekennzeichnet,** daß zur Beeinflussung von (rechtsschraubig) linksdrehend zirkular-/elliptisch-polarisierten Gruppen-/Raumwellen des Senders die Antennenspiralabschnitte in Fortpflanzungsrichtung der Phasen-/Strahlungsfront gesehen, im Uhrzeigersinn sich öffnend ausgerichtet sind, wobei zirkular-/elliptisch-polarisierte Feldkomponenten in einem dem Sender abgewandten Strahlungsbereich der Antenne gelöscht sind.

6. Anordnung nach einem der Ansprüche 1 bis 5, **dadurch gekennzeichnet,** daß die Antennenspiralabschnitte (14, 15) beidseitig quer zu der Antennen-Zentrumachse (18) mit einem zu den Abschnitten isolierten, linear-polarisierte Wellen reflektierenden Material (6) abgedeckt sind.

7. Anordnung nach einem der Ansprüche 1 bis 5, **dadurch gekennzeichnet,** daß die Anordnung (74) zwei gleiche, einen Sender (2) mit einer strahlenden Linie (21) umgebende Antennen (1) umfaßt, wobei die Antennen jeweils mit ihrer zu der Antennen-Zentrumachse (18) senkrechten Antennenfläche (100) parallel zueinander ausgerichtet sind, sich jeweils mit einer in der Antennenfläche liegenden Antennenlängsachse (180) entlang der strahlenden Linie (21) erstrecken und jeweils die Antennen-Spiralabschnitte (14, 15), von dem Sender aus gesehen, im Gegen-Uhrzeigersinn sich öffnend ausgerichtet und auf den dem Sender abgewandten Antennenseiten (W2) mit einem zu den Spiralabschnitten isolierten, linear-polarisierte Wellen reflektierenden Material (6) abgedeckt sind.

8. Anordnung nach Anspruch 6, **dadurch gekennzeichnet,** daß mehrere gleiche Antennen (1) mit gleichem Öffnungssinn sämtlicher Antennen-Spiralabschnitte neben-/hintereinander mit parallelen Antennenlängsachsen (180) und/oder mit parallelen Antennenquerachsen (130) angeordnet sind.

9. Anordnung nach einem der Ansprüche 1 bis 8, **dadurch gekennzeichnet,** daß die Anordnung (7) einen mindestens eine Antenne (1) lagernden Tragkörper (70) wie ein Lager, ein Gehäuse, ein Gestell oder einen Rahmen umfaßt, an und/oder mit dem eine Antenne um ihr Zentrum (13) drehbar (D) und/oder entlang wenigstens einer Antennenachse verschiebbar (d) angeordnet ist.

10. Verfahren zum Ermitteln und/oder Beeinflussen von elek tromagnetischen Raumwellen und/oder einer Gruppe solcher Wellen, die von Sendern oder Strahlern (2) wie Mikrowellen erzeugenden Einrichtungen, Körpern, Resonanzquellen oder -strukturen, z.B. geologischen Spalten oder Verwerfungen, Strömungen fließender Medien (z.B. Wasser) od.dgl. abgestrahlt werden, wobei die elektromagnetischen Raumwellen oder die Gruppe solcher Wellen des Senders mit einem Empfangssystem (3) aufgesucht und eine Antenne (1) zur Beeinflussung der elektromagnetischen Gruppen-/Raumwellen in den Strahlungsbereich gebracht und ausgerichtet wird, **dadurch gekennzeichnet,** daß

a) als Empfangssystem (3) ein solches für elektromagnetische zirkular- oder elliptischpolarisierte Wellen (F, FO) verwendet wird,

b) mit dem Empfangssystem (3) eine elektromagnetische Phasen- oder Strahlungsfront (200) mit höchster Rauschintensität pro Raumvolumeneinheit aufgesucht wird,

c) als Antenne (1) wenigsten eine solche mit großer Bandbreite verwendet wird, die mindestens zwei um eine Antennen-Zentrum- oder Strahlungsachse (18) sich sämtlich jeweils links- bzw. rechtsdrehend öffnende, im Abstand und isoliert zueinander angeordnete spiralförmige Abschnitte oder Arme (14, 15) für elektromagnetische zirkular- oder elliptisch-polarisierte Wellen sowie kugelflächenoder kreisflächenförmige, mit im Bereich des Antennenzentrums (13) liegenden inneren Spiralabschnitten (131, 132) verbundene Antennen-Dipolabschnitte (133, 134) aufweist,

d) die Antenne (1) derart angeordnet und ausgerichtet wird, daß das Antennenzentrum (13), in einer Phasen-/Strahlungsfront (200) mit höchster Rauschintensität zu liegen kommt, und anschließend

e) die Antenne (1) durch Drehung (D) um das Antennenzentrum (13) und/oder Verschiebung (d) des Antennenzentrums (13) entlang einer Phasen-/Strahlungsfront (200) mit höchster Rauschintensität in eine die elektromagnetische zirkular- oder elliptisch-polarisierte Raumwellen oder Raumwellengruppe (F, FO) beeinflussende Position (7) gebracht wird.

11. Verfahren nach Anspruch 10, **dadurch gekennzeichnet,** daß zum Aufsuchen von zirkular-/elliptisch-polarisierten elektromagnetischen Sender-Gruppen-/Wellen (F, FO) ein an sich bekanntes Empfangssystem mit rauscharmem Empfänger (3) und mit rauscharmer Antenne, insbesondere mit einer im Antennenzentrum eine Wendelantenne aufweisenden Rotationsparabolantenne verwendet wird.

12. Verfahren nach Anspruch 10, **dadurch gekennzeichnet,** daß zum Aufsuchen von elektromagnetischen zirkular-/elliptisch-polarisierten Sender-Gruppen-/Wellen (F, FO) für das Empfangssystem (3) eine an sich bekannte, auf das menschliche Nerven-/Muskelsystem wirkende Hoch- oder Höchstfrequenzantenne, insbesondere eine V-förmige Antenne oder eine Lecher-Antenne mit Kurzschlußschieber verwendet wird, die insbesondere über einem Nord- oder Süd-Pol eines Magneten oder über dem Plus- oder Minus-Pol einer Batterie zur Feststellung einer Empfangsebene für ein magnetisches Feld bzw. für ein elektrisches Feld geeicht wird.

13. Verfahren nach einem der Ansprüche 10 bis 12, **dadurch gekennzeichnet,** daß eine Antennenlängsachse (180), auf der äußere Spiralabschnitenden (181, 182) münden, im wesentlichen parallel zu einer Phasen-/Strahlungsfront (200) höchster Rauschintensität ausgerichtet und eine senkrecht zu der Antennen-Zentrumachse (18) liegende Antennenfläche (100) quer, insbesondere senkrecht zu einer Fortpflanzungsrichtung (S) der Phasen-/Strahlungsfront angeordnet wird.

14. Verfahren nach einem der Ansprüche 10 bis 13, **dadurch gekennzeichnet,** daß zur Beeinflussung von (linksschraubig (L)) rechtsdrehend (P) zirkular-/elliptisch-polarisierten Gruppen-/Raumwellen des Senders (2) die Antennenabschnitte (14, 15), in einer Fortpflanzungsrichtung (S) der Phasen-/Strahlungsfront (200) gesehen, im Gegen-Uhrzeigersinn sich öffnend ausgerichtet werden, um zirkular-/elliptisch-polarisierte Feldkomponenten in einem dem Sender (2) abgewandten Strahlungsbereich (WI) der Antenne (1) zu löschen.

15. Verfahren nach Anspruch 14, **dadurch gekennzeichnet,** daß Antennenspiralabschnitte (14, 15) beidseitig quer zu der Antennen-Zentrumachse (18) mit einem zu den Abschnitten isolierten, linear-polarisierte Wellen reflektierenden Material (6) abgedeckt werden.

16. Verfahren nach einem der Ansprüche 10 bis 13, **dadurch gekennzeichnet ,** daß ein Sender (2) mit einer (linksschraubig (L)) rechtsdrehend (P) zirkular-/elliptisch-polarisierte Grup pen-/Raumwellen abstrahlenden Linie (21) mit zwei gleichen Antennen (1) derart umgeben wird, daß die Antennen mit jeweils zu der Antennen-Zentrumachse (18) senkrechter Antennenfläche (100) parallel zueinander, jeweils mit in der Antennenfläche liegender Antennenlängsachse (180) entlang der strahlenden Linie sowie jeweils , von der strahlenden Linie aus gesehen, mit im Gegen-Uhrzeigersinn sich öffnenden Spiralabschnitten (14, 15) ausgerichtet werden, wobei die Spiralabschnitte auf den der strahlenden Linie abgewandten Antennenseiten (W2) mit einem zu den Abschnitten isolierten, linear-polarisierte Wellen reflektierenden Material (6) abgedeckt werden.

17. Antenne für den Aufbau einer Anordnung (7) und/oder für die Ausführung eines Verfahrens zum Ermitteln und/oder Beeinflussen von elektromagnetischen Raumwellen und/oder einer Gruppe solcher Wellen, die von Sendern oder Strahlern (2) wie Mikrowellen erzeugenden Einrichtungen, Körpern, Resonanzquellen oder -strukturen, z.B. geologischen Spalten oder Verwerfungen, Strömungen fließender Medien (z.B. Wasser) od.dgl. abgestrahlt werden, **dadurch gekennzeichnet,** daß die Antenne (1) mindestens zwei um eine Antennen-Zentrum- oder -Strahlungsachse (18) sich sämtlich jeweils links- bzw. rechtsdrehend öffnende, im Abstand und isoliert zueinander angeordnete Spiralarme (14, 15) umfaßt, deren innere Spir-'alarmenden (131, 132) im Bereich des Antennenzentrums (13) liegen und daß mit mindestens zwei inneren Spiralarmenden (131, 132) jeweils ein kreis- oder kugelförmiges Dipol-Antennenelement (133, 134) zur Ausbildung einer mit der Spiralarmantenne integrierten Dipolantenne verbunden ist.

18. Antenne nach Anspruch 17, **dadurch ge kennzeichnet,** daß die im Bereich des Antennenzentrums (13) liegenden inneren Spiralarmenden (131, 132) von einer Antennenquerachse (130) ausgehen, die im Antennenzentrum senkrecht auf der Antennen-Zentrumachse (18) steht.

19. Antenne nach einem der Ansprüche 17 oder 18, **dadurch gekennzeichnet,** daß äußere Spiralarmenden (181, 182) auf einer Antennenlängsachse (180) enden, die im Antennenzentrum (13) senkrecht auf der Antennen-Zentrumachse (18) steht.

20. Antenne nach einem der Ansprüche 17 bis 19, **dadurch gekennzeichnet,** daß die Spiralarme (14, 15) und/oder die Dipol-Antennenelemente (133, 134) aus einem einen elektrischen Widerstand aufweisenden Material, vorzugsweise aus Kohlenstoff oder Graphit bestehen.

21. Antenne nach einem der Ansprüche 17 bis 20, **dadurch gekennzeichnet,** daß die Spiralarme (14, 15) flächenförmig in oder an einer senkrecht zu der Antennenzentrum-Achse (18) liegenden ebenen Fläche (100) eines Antennenkörpers (10) ausgebildet sind.

22. Antenne nach Anspruch 21, **dadurch gekennzeichnet,** daß der Antennenkörper (10) blattförmig mit im Verhältnis zu seinen Flächenmaßen sehr kleiner Dicke ausgebildet ist.

23. Antenne nach Anspruch 21 oder 22, **dadurch gekennzeichnet,** daß der Antennenkörper (10) wenigstens an einer ebenen Fläche (100) eine abziehbare, eine Haft- oder Klebeschicht freilegende Abdeckschicht wie eine Folie aufweist.

24. Antenne nach einem der Arsprüche 17 bis 23, **dadurch gekennzeichnet,** daß sich die Spiralarme (14, 15) in oder parallel zu einer Fläche erstrecken, deren Flächeninhalt kleiner als 600 mm$^2$ ist.

25. Antenne nach einem der Arsprüche 17 bis 24, **dadurch gekennzeichnet,** daß der Durchmesser (S) oder die Dicke eines jeden Spiralarmes (14, 15) in Spiralöffnungsrichtung zunehmend ausgebildet ist.

26. Antenne nach Anspruch 25, **dadurch gekennzeichnet,** daß sie als logarithmische Spiralantenne ausgebildet ist.

27. Antenne nach einem der Ansprüche 16 bis 26, **dadurch gekennzeichnet,** daß koaxiale Achsen der Dipol-Antennenelemente (133, 134) parallel und symmetrisch zu der Spiralantennen-Zentrumachse (18) liegen.

28. Antenne nach einem der Ansprüche 16 bis 27, **dadurch gekennzeichnet,** daß die Spiralarme (14, 15) sowie die Dipol-Antennenelemente (133, 134) quer zu der Antennenzentrum-Achse (18) wenigstens einseitig mit einem zu den Armen und Dipolelementen isolierten, linearpolarisierte Wellen reflektierenden Material (6) vorzugsweise in Form einer Metallschicht z.B. aus Aluminium abgedeckt sind.

29. Antenne nach Anspruch 28, **dadurch gekennzeichnet,** daß die Spiralarme (14, 15) und die Dipol-Antennenelemente (133, 134) mindestens einlagig in eine Metallfolie, insbesondere in eine Aluminiumfolie eingebettet oder mit dieser umwickelt sind.

Fig.2

Fig.3a

Fig.3

0280644

Fig.1

Fig.5a

Fig.5

Fig.5b

Fig.4a

133        1

14    5        6

13

100        15

134

72

6        70

W1

a

5        F        a

S

Fig.4        2

0280644

Fig. 6